# EUROPÄISCHE PATENTANMELDUNG

(11) **EP 1 400 226 A1**
(43) Veröffentlichungstag der Anmeldung: **24.03.2004**
(21) Anmeldenummer: 03010858.3
(22) Anmeldetag: 15.05.2003
(51) Int. Cl.: A61F 13/514, A61F 13/15

(54) **Slipeinlage**

(30) Priorität: 18.09.2002 DE 10243109
(71) Anmelder: Hygiene Oederan Vertriebsgesellschaft mbH, 45481 Mülheim an der Ruhr (DE)
(72) Erfinder: Schmidt, Lothar, 09569 Oederan (DE); Nennstiel, Martina, 46147 Oberhausen (DE)
(74) Vertreter: COHAUSZ DAWIDOWICZ HANNIG & PARTNER

(57) **Zusammenfassung**

Die Erfindung betrifft eine Slipeinlage mit einer Flüssigkeit aufnehmenden Saugschicht (1), auf deren Vorderseite eine Flüssigkeit durchlässige Lage (2) und auf deren Rückseite eine Flüssigkeit undurchlässige Lage (3) befestigt ist. Auf der undurchlässigen Lage ist rückseitig eine zusätzliche Lage (5) lösbar befestigt, deren Färbung zumindest außenseitig unterschiedlich ist von der außenseitigen Färbung der undurchlässigen Lage (3).

## Beschreibung

Die Erfindung betrifft eine Slipeinlage mit einer Flüssigkeit aufnehmenden Saugschicht, auf deren Vorderseite eine Flüssigkeit durchlässige Lage und auf deren Rückseite eine Flüssigkeit undurchlässige Lage befestigt ist.

Slipeinlagen sind als täglicher Wäscheschutz in der Damenhygiene etabliert. Sie werden in den Damenslip eingelegt und halten am Stoff des Slips aufgrund eines Haftklebers.

Früher gab es ausschließlich weiße Slipeinlagen. Verbraucherinnen, die neben weißer auch schwarze Unterwäsche tragen, hatten den Anspruch, schwarze Slipeinlagen zu verwenden. Es sollte verhindert werden, dass die weiße Farbe durch die Unterwäsche sichtbar wird. Aus diesen Ansprüchen heraus wurden schwarze Slipeinlagen entwickelt und angeboten.

Diese neue Produktvariante wurde von den Verbraucherinnen gut angenommen. Nachteilig ist, dass die beiden Produktvarianten grundsätzlich eine doppelte Bevorratung, nämlich von weißen und schwarzen Slipeinlagen, erfordert. Dies ist insbesondere auf Reisen ein Problem. Die Möglichkeit, Verpackungen mit gemischt sortierten weißen und schwarzen Slipeinlagen anzubieten, ist keine wirkliche Alternative wegen der problematischen Definition eines geeigneten Mischungsverhältnisses.

Aufgabe der Erfindung ist es, eine Slipeinlage der eingangs genannten Art so zu verbessern, dass dieselbe Slipeinlage wahlweise eine schwarze oder eine weiße zum Slip hingewandte Rückseite aufweist.

Diese Aufgabe wird erfindungsgemäß dadurch gelöst, dass auf der undurchlässigen Lage rückseitig eine zusätzliche Lage lösbar befestigt ist, deren Färbung zumindest außenseitig unterschiedlich ist von der außenseitigen Färbung der undurchlässigen Lage.

In einer Alternative wird vorgeschlagen, dass die Saugschicht beidseitig saugfähig ist und zu beiden Seiten mit einer lösbaren undurchlässigen Lage bedeckt ist, die jeweils zumindest außenseitig eine unterschiedliche Färbung aufweisen.

Bei einer weiteren Alternative kann die Saugschicht beidseitig saugfähig sein, auf jeder Seite eine andere Färbung aufweisen und zu beiden Seiten mit einer lösbaren, undurchlässigen transparenten Lage bedeckt sein.

Auch wird in einer Alternative vorgeschlagen, dass die Saugschicht beidseitig saugfähig ist und an ihren Seitenrändern klappenförmige undurchlässige Lagen befestigt sind, die jeweils auf ihren beiden Seiten unterschiedlich gefärbt sind.

Durch geeignete Manipulation der erfindungsgemäßen Slipeinlage, können die Verbraucherinnen individuell entscheiden, ob die Slipeinlage eine weiße oder eine schwarze Oberfläche zur Wäscheseite hin besitzt.

Damit wird auf konstruktiv einfache Weise das Problem der doppelten Vorratshaltung, oder auch das Problem eines geeigneten Mischungsverhältnisses von Kombinationsverpackungen erfolgreich gelöst. Hierbei ist die Handhabung der Farbwahl besonders einfach.

Vorzugsweise wird vorgeschlagen, dass die lösbare(n) Lage(n) außenseitig einen dauernd klebfähigen Haftkleber aufweist (aufweisen), der mit einer abziehbaren Fläche insbesondere Papier oder Folie bedeckt ist.

Grundsätzlich besteht die Lösung der Aufgabenstellung darin, dass jedes Produkt mit mehrfarbigen Komponenten ausgerüstet ist. Diese farbigen Komponenten werden in geeigneter Weise zueinander angeordnet. Dieser Grundidee folgend bieten sich mehrere Möglichkeiten.

Ausführungsbeispiele der Erfindung sind in den Zeichnungen schematisch dargestellt und werden im folgenden näher beschrieben. Es zeigen
- Fig. 1: eine erste Ausführung mit einer zusätzlichen die Rückseite der Saugschicht abdeckenden lösbaren Lage unterschiedlicher Färbung zur Saugschichtrückseite,
- Fig. 2: eine zweite Ausführung mit zusätzlichen lösbaren Lagen auf beiden Seiten der Saugschicht,
- Fig. 3: eine dritte Ausführung mit klappenförmiger zusätzlicher Lage.

In den Zeichnungen der Ausführungsbeispiele sind die Formen und Außenränder der Schichten und Lagen vereinfacht rechteckig und damit ohne Rundungen dargestellt.

Bei allen im folgenden beschriebenen Ausführungen ist gemeinsam, dass jeweils eine Saugschicht 1 vorhanden ist, die zumindest auf einer Seite mit einer nicht entfernbaren flüssigkeitsdurchlässigen Lage 2 bedeckt ist. Diese Seite wird im folgenden die "Vorderseite" genannt.

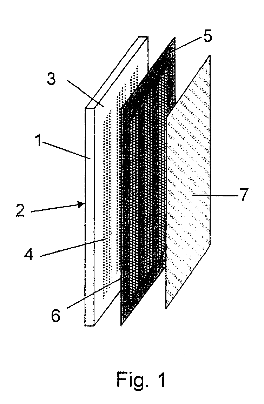

Bei bekannten Slipeinlagen ist die Rückseite der Saugschicht 1 durch eine undurchlässige Lage 3 bedeckt, die einen dauernd klebfähigen Haftkleber 4 insbesondere in Streifenform aufweist. In gleicher Weise ist auch die Saugschicht 1 beim ersten Ausführungsbeispiel nach Fig. 1 ausgeführt. Während aber im Stand der Technik der Haftkleber 4 der Lage 3 durch eine Abdeckfläche 7 direkt bedeckt ist, liegt im ersten Ausführungsbeispiel zwischen der Lage 3 und der Abdeckfläche 7 eine zusätzliche lösbare Lage 5. Diese Lage 5 ist lösbar auf der Lage 3 durch den Haftkleber 4 befestigt und weist an ihrer Rückseite einen Haftkleber 6 insbesondere streifenförmig auf, der durch die lösbare Abdeckfläche 7 bedeckt ist.

Im Ausführungsbeispiel nach Fig. 1 ist die Rückseite der Saugschicht 1 und damit die undurchlässige Lage 3 in weißer Färbung und zumindest die Außenseite der zusätzlichen lösbaren Lage 5 in schwarzer Färbung. Will die Verbraucherin, dass die Rückseite der Slipeinlage eine weiße Färbung hat, so zieht sie sowohl die Abdeckfläche 7 als auch die Lage 5 von der Rückseite der Saugschicht 1 ab und die Slipeinlage ist durch den Haftkleber 4 im Slip befestigbar.

Soll dagegen die Rückseite der Slipeinlage eine schwarze Färbung aufweisen, so wird die Lage 5 auf der Saugschicht 1 belassen und nur die Abdeckfläche 7 abgezogen.

Alternativ kann auch die Rückseite der Saugschicht 1 und damit die Lage 3 schwarz gefärbt sein und zumindest die Rückseite der Lage 5 eine weiße Färbung besitzen. Auch in diesem Fall kann durch das Abziehen der Lage 5 oder durch das Belassen der Lage 5 auf der Saugschicht 1 die Färbung gewählt werden.

In diesem ersten Ausführungsbeispiel als auch bei den folgenden Ausführungsbeispielen wird stets von einer weißen und schwarzen Färbung gesprochen. Hierunter ist gemeint, dass das Material nicht unbedingt weiß oder schwarz eingefärbt sein muss, sondern dass das Material auch selber von sich aus die jeweilige Farbe aufweisen kann, ohne eingefärbt sein zu müssen. Ferner können statt einer weißen und einer schwarzen Färbung auch andere Farben gewählt sein, so zum Beispiel eine helle oder dunkle Farbe bzw. Beige oder eine braune Farbe. Wenn also von den Farben weiß und schwarz gesprochen wird, so dies nur zur Vereinfachung der Beschreibung.

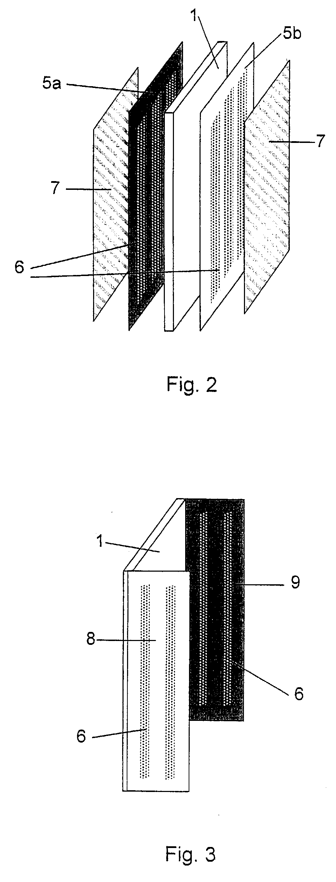

Beim Ausführungsbeispiel nach Fig. 1 ist die Saugschicht auf der Vorderseite flüssigkeitsdurchlässig (durchlässige Lage 2) und auf der Rückseite flüssigkeitsundurchlässig (undurchlässige Lage 3). Das Ausführungsbeispiel nach Fig. 2 unterscheidet sich hiervon unter anderem dadurch, dass die Saugschicht 1 vorderseitig und rückseitig flüssigkeitsdurchlässig ist, das heißt das saugende Material der Schicht 1 ist vorderseitig und rückseitig mit unentfernbaren flüssigkeitsdurchlässigen Schichten bedeckt. Die Saugschicht 1 ist somit beidseitig saugfähig und auf beide Seiten ist jeweils eine zusätzliche flüssigkeitsundurchlässige Lage 5a, 5b lösbar befestigt. Die lösbaren Lagen 5a, 5b weisen hierbei beidseitig insbesondere in Streifenform einen dauernd klebfähigen Haftkleber 6 auf, wobei diese beiden Lagen 5a, 5b wiederum durch Schutzflächen 7 abgedeckt sind, die die Klebflächen bedecken.

Die eine zusätzliche lösbare Lage 5a ist vorzugsweise zumindest außenseitig schwarz gefärbt und die andere Lage 5b außenseitig weiß gefärbt. Je nach Bedarf wird die schwarze 5a oder weiße Befestigungslage 5b abgezogen, wonach dann die auf der Saugschicht 1 verbleibende Lage die Rückseite der Slipeinlage bildet. Von dieser Lage wird dann die Schutzfläche 7 abgezogen, um eine Befestigung im Slip zu erlauben.

In einer weiteren Alternative, die der Ausführung nach Fig. 2 ähnlich ist, sind die Lagen 5a, 5b transparent ausgeführt und die Außenseiten der Saugschicht 1 unterschiedlich gefärbt. So kann beispielsweise wiederum eine Seite schwarz und die andere Seite weiß gefärbt sein. Vor Gebrauch wird dann die Lage 5a oder 5b auf der Seite abgezogen, die die Vorderseite bilden soll, so dass dann die Saugschichtseite die Slipeinlagenrückseite bildet, deren Färbung dort gewünscht ist.

Bei der Ausführung nach Fig. 3 wird die zusätzliche lösbare Lage durch zwei klappenförmige Lagen bzw. Flügel 8, 9 gebildet, die seitlich an beiden Längsseiten der Saugschicht 1 befestigt sind und die zur einen oder anderen Seite der Saugschicht 1 geklappt werden können. Hierbei ist die Saugschicht 1 auf beiden Seiten saugfähig und die Lagen 8, 9 besitzen auf beiden Seiten unterschiedliche Färbungen, insbesondere Schwarz und Weiß und tragen dort jeweils dauernd klebfähige Haftkleber 6 insbesondere in Streifenform.

Die beiden Klappen bzw. Flügel 8, 9 werden wahlweise auf die eine oder andere Seite der Saugschicht 1 in der Weise geklappt, dass dann die schwarze oder weiße Färbung zu oberst liegt, um dann die Rückseite der Slipeinlage zu bilden.

## Patentansprüche

1. Slipeinlage mit einer Flüssigkeit aufnehmenden Saugschicht (1), auf deren Vorderseite eine Flüssigkeit durchlässige Lage (2) und auf deren Rückseite eine Flüssigkeit undurchlässige Lage (3) befestigt ist, **dadurch gekennzeichnet, dass** auf der undurchlässigen Lage (3) rückseitig eine zusätzliche Lage (5) lösbar befestigt ist, deren Färbung zumindest außenseitig unterschiedlich ist von der außenseitigen Färbung der undurchlässigen Lage (3). (Fig. 1)

2. Slipeinlage mit einer Flüssigkeit aufnehmenden Saugschicht (1), auf deren Vorderseite eine Flüssigkeit durchlässige Lage (2) und auf deren Rückseite eine Flüssigkeit undurchlässige Lage befestigt ist, **dadurch gekennzeichnet, dass** die Saugschicht (1) beidseitig saugfähig ist und zu beiden Seiten mit einer lösbaren undurchlässigen Lage (5a, 5b) bedeckt ist, die jeweils zumindest außenseitig eine unterschiedliche Färbung aufweisen. (Fig. 2).

3. Slipeinlage mit einer Flüssigkeit aufnehmenden Saugschicht (1), auf deren Vorderseite eine Flüssigkeit durchlässige Lage (2) und auf deren Rückseite eine Flüssigkeit undurchlässige Lage befestigt ist, **dadurch gekennzeichnet, dass** die Saugschicht (1) beidseitig saugfähig ist, auf jeder Seite eine andere Färbung aufweist und zu beiden Seiten mit einer lösbaren, undurchlässigen transparenten Lage (5a, 6b) bedeckt ist. (ähnlich Fig. 2)

4. Slipeinlage mit einer Flüssigkeit aufnehmenden Saugschicht (1), auf deren Vorderseite eine Flüssigkeit durchlässige Lage (2) und auf deren Rückseite eine Flüssigkeit undurchlässige Lage befestigt ist, **dadurch gekennzeichnet, dass** die Saugschicht (1) beidseitig saugfähig ist und an ihren Seitenrändern klappenförmige undurchlässige Lagen (8, 9) befestigt sind, die jeweils auf ihren beiden Seiten unterschiedlich gefärbt sind. (Fig. 3)

5. Slipeinlage nach einem der vorherigen Ansprüche, **dadurch gekennzeichnet, dass** die lösbare(n) Lage(n) außenseitig einen dauernd klebfähigen Haftkleber aufweist (aufweisen), der mit einer abziehbaren Fläche (7) insbesondere Papier oder Folie bedeckt ist.
